(19) Europäisches Patentamt
European Patent Office
Office européen des brevets

(11) Numéro de publication: **0 143 059**
**A1**

(12) **DEMANDE DE BREVET EUROPEEN**

(21) Numéro de dépôt: **84402392.9**

(22) Date de dépôt: **23.11.84**

(51) Int. Cl.⁴: **C 12 Q 1/68**
C 12 N 15/00, C 07 H 21/04
C 12 N 1/20
//(C12N1/20, C12R1:19)

(30) Priorité: **24.11.83 FR 8318785**
**31.01.84 FR 8401480**

(43) Date de publication de la demande:
**29.05.85 Bulletin 85/22**

(84) Etats contractants désignés:
**AT BE CH DE FR GB IT LI LU NL SE**

(71) Demandeur: **INSTITUT PASTEUR**
**25-28, rue du Docteur Roux**
**F-75724 Paris Cedex 15(FR)**

(71) Demandeur: **CENTRE NATIONAL DE LA RECHERCHE SCIENTIFIQUE (C.N.R.S.)**
**15, Quai Anatole France**
**F-75007 Paris(FR)**

(72) Inventeur: **Avrameas, Stratis**
**1, rue Sarasate**
**F-75015 Paris(FR)**

(72) Inventeur: **Danchin, Antoine**
**60, rue de Reuilly**
**F-75012 Paris(FR)**

(72) Inventeur: **Ternynck, Thérèse**
**39, Boulevard Garibaldi**
**F-75015 Paris(FR)**

(72) Inventeur: **Traincard, François**
**241, rue de la Croix Nivert**
**F-75015 Paris(FR)**

(74) Mandataire: **Gutmann, Ernest et al,**
**Cabinet Plasseraud 84, rue d'Amsterdam**
**F-75009 Paris(FR)**

(54) **Sonde non radioactive et procédé pour la caractérisation d'une séquence nucléotidique déterminée et moyens pour la production de cette sonde non radioactive.**

(57) L'invention concerne une sonde non radioactive pour la détection par hybridation d'une séquence déterminée de nucléotides et comportant à titre de constituant essentiel un acide nucléique contenant lui-même un insérat comprenant une séquence complémentaire de ladite séquence déterminée de nucléotides. Cette sonde est caractérisée en ce qu'au moins une partie d'au moins l'un des nucléotides constitutifs de son acide nucléique est remplacée par des bases modifiées reconnaissables par les polymérases et comportant un groupe comprenant au plus deux atomes de carbone et, de préférence, au moins un groupe halogène.

EP 0 143 059 A1

Sonde non radioactive et procédé pour la caractérisation d'une séquence nucléotidique déterminée et moyens pour la production de cette sonde non radioactive.

---

L'invention est relative à une nouvelle sonde spécifique marquée non radioactive et à un procédé pour la détection par hybridation de la présence et, éventuellement, de la caractérisation d'une séquence nucléotidique déterminée au sein d'une composition à base d'acides nucléiques (ADNs, cADNs, ARNs), cette sonde marquée non radioactive contenant elle-même une séquence nucléotidique complémentaire de la précédente et susceptible de s'hybrider dans les conditions appropriées avec ladite séquence nucléotidique déterminée à détecter. L'invention est également relative à un procédé et à des moyens pour la fabrication d'une telle sonde non radioactive. L'invention concerne enfin les applications dans de nombreux domaines de cette nouvelle sonde non radioactive, ces applications ayant en général toutes en commun l'isolement à partir d'un échantillon biologique de sa teneur en acides nucléiques et la détection de la présence ou non, au sein de ces acides nucléiques (ADN, ARN ou les deux à la fois) de la séquence nucléotidique déterminée par hybridation avec la séquence complémentaire contenue dans la sonde selon l'invention.

Les sondes d'ADN spécifiques, encore à ce jour les plus couramment utilisées pour repérer une hybridation ADN-ADN ou ADN-ARN dans les expériences d'hybridation effectuées soit in vitro, soit in situ, sont marquées avec des isotopes et mettent en jeu une détection autoradiographique.

Les principaux désavantages de la détection autoradiographique de l'hybridation sont les dangers

encourus par l'expérimentateur lorsqu'il manipule des produits radioactifs, le faible pouvoir de résolution, l'imprécision de la localisation, la longueur du temps d'exposition des films (de quelques heures à plusieurs semaines) et l'instabilité des sondes due à la décomposition radiolytique.

Pour remédier à cette difficulté, il a déjà été proposé de remplacer le marquage radioactif par d'autres modes de marquage. On rappellera en particulier la technique décrite pour la première fois dans le brevet français n° 78 10975 déposé le 13 avril 1978.

Le procédé décrit dans le brevet sus-visé pour la détection de la présence éventuelle ou de caractérisation d'une séquence ou fragment déterminé d'acide nucléique, notamment d'un gène, voire de l'acide nucléique entier au sein d'un échantillon d'acides nucléiques complexe, par mise en contact de l'échantillon, le cas échéant après dénaturation préalable de l'acide nucléique étudié, avec une sonde comprenant un acide nucléique complémentaire, susceptible de s'hybrider avec la séquence d'acide nucléique ou l'acide nucléique recherché, est caractérisé en ce que la sonde utilisée est une sonde modifiée chimiquement par couplage ou en vue de son couplage avec une enzyme, de préférence postérieurement à la réaction d'hybridation, l'éventuelle présence de la séquence d'acide nucléique ou de l'acide nucléique cherché étant ensuite révélable par l'action du produit d'hybridation formé à partir de la sonde et de la séquence ou de l'acide nucléique recherché, sur un substrat de l'enzyme.

Avantageusement, l'enzyme est choisie selon sa capacité à agir sur un substrat chromogène, ce qui permet de doser, par analyse optique ou analogue, le taux de transformation du substrat, taux qui est alors corrélable à la présence ou non de la séquence d'acide

nucléique ou de l'acide nucléique recherché dans l'échantillon initial.

Dans un mode de mise en oeuvre préféré de l'invention du brevet, la sonde est modifiée par un groupe chimique susceptible de former un complexe stable avec l'enzyme ou une molécule elle-même liée de façon stable à l'enzyme. Avantageusement, le susdit groupe chimique et la susdite molécule sont respectivement constitués par la biotine et l'avidine ou vice versa, l'enzyme étant elle-même constituée par exemple par la bêta-galactosidase, la glucose-oxydase, phosphatase alcaline,péroxydase.

La détection ou "révélation" de la sonde hybridée peut mettre en oeuvre des moyens distincts de l'enzyme, par exemple des techniques d'immunofluorescence.

Le couplage provoqué entre la sonde hybridée et l'enzyme ou autres moyens de révélation distincts mis en oeuvre dans le procédé rappelé ci-dessus peut être réalisé soit directement (par exemple par l'intermédiaire de la réaction biotine-avidine ou d'une réaction antigène-anticorps), soit indirectement, par exemple dans une séquence de réactions comprenant par une première mise en contact de la sonde hybridée porteuse de groupes hapténiques avec des anticorps anti-haptène, puis une seconde mise en contact de la sonde hybridée qui retient alors des anticorps anti-haptène, avec des immunoglobulines ou autres molécules affines dirigées contre des immunoglobulines, elles-mêmes marquées en application de l'une des techniques non radioactives qui a été rappelée ou par des techniques équivalentes. Il n'est pas besoin d'insister sur les conditions dans lesquelles ces techniques peuvent être mises en oeuvre. Elles sont bien connues. Si besoin, on se reportera aussi à la demande de brevet européen n° 0063869-A3 dans laquelle elles ont été rappelées.

Il faut cependant remarquer aussi que cette demande de brevet européen, entre autres caractéristiques, étend aussi le champ des possibilités de marquage de sondes nucléiques non radioactives et décrit des sondes d'acides nucléiques dans lesquelles certaines bases portent des substituants qui, selon les enseignements de cette demande de brevet, doivent comporter au moins trois atomes de carbone,et de préférence même au moins cinq atomesde carbone, ces groupes étant choisis parmi ceux qui peuvent être détectés par une molécule, notamment un polypeptide témoignant d'une grande affinité pour ces groupes, et ce lorsque la sonde forme un duplex en double hélice avec une séquence ribonucléique ou désoxyribonucléique complémentaire d'un fragment de nucléotides contenu dans la sonde.

Les techniques qui viennent d'être rappelées et qui mettent toutes deux en oeuvre des sondes non radioactives ou, pour la commodité du langage ci-après des "sondes froides" (par opposition aux "sondes chaudes" que constituent les sondes marquées avec des isotopes radioactifs), présentent des avantages importants par rapport aux "sondes chaudes", ne serait-ce qu'au niveau de la plus grande sécurité des expérimentateurs qui les mettent en oeuvre. Mais il reste toujours encore nécessaire de les perfectionner pour les rendre à la fois plus efficaces et plus sensibles.

En outre, il y a lieu de remarquer le caractère encore à la fois complexe et coûteux de la réalisation du marquage proprement dit des sondes en question : nécessité d'abord de synthétiser des nucléotides modifiés dans les conditions qui ont été décrites au brevet, puis incorporation <u>in vitro</u> de ces nucléotides modifiés, par exemple des biotinyl-nucléotides , dans les chaînes bicaténaires des acides nucléiques à marquer par une technique réalisant des ouvertures et des réparations localisées de l'une des chaînes au moyen d'une polymérase (technique dite de "nick-translation".

Il a également été proposé dans la demande de brevet européen de polymériser le nucléotide modifié précédemment obtenu, par exemple le biotinyl-dUTP, en présence d'une matrice ("template") comportant une extrémité terminale de poly-A, en présence d'une ARN-ligase.

L'invention a donc pour but de fournir des sondes froides qui répondent aux objectifs suivants :
- les sondes doivent s'hybrider facilement aux acides nucléiques,
- la modification de certaines de leurs bases nucléotidiques doit être minimale, de façon que ces sondes restent capables de s'hybrider à des brins d'acides nucléiques complémentaires,
- la sensibilité de la méthode doit être au moins égale à celle obtenue avec les sondes radioactives.

L'invention a encore pour but des moyens et techniques de fabrication permettant l'obtention rapide et à relativement peu de frais de ces sondes ou plus généralement d'ADNs marqués.

D'une façon générale, la sonde froide selon l'invention pour la détection d'une séquence déterminée de nucléotides par hybridation, et qui comporte elle-même un insérat comprenant une séquence complémentaire de ladite séquence de nucléotides, est caractérisée en ce qu'au moins une partie des bases d'au moins l'un des types de nucléotides constitutifs de son acide nucléique est remplacée par des bases modifiées reconnaissables par les polymérases, et comportant un groupe comprenant au plus deux atomes de carbone. De préférence, ce groupe comporte au moins un halogène ou est constitué par un halogène.

D'une façon générale, on peut mettre en oeuvre toute base modifiée répondant aux conditions qui précèdent et qui n'interfèrent pas avec la capacité d'appariement WATSON-CRICK de l'acide nucléique ainsi modifié avec un acide nucléique complémentaire naturel ou contenant des bases modifiées semblables.

De préférence, les bases modifiées sont dérivées des bases nucléotidiques naturelles dont la modification réside dans une substitution par le susdit groupe, en position 5, lorsqu'il s'agit d'une base pyrimidine et en position 7 ou en position 8, ou dans les deux positions à la fois, d'une base déazapurine ou purine.

Les sondes préférées sont celles dans lesquelles les nucléosides modifiés sont des groupes 5-halogéno-uridine ou 5-halogéno-déoxy-uridine, et plus particulièrement encore celles dans lesqueles ces bases modifiées sont des groupes 5-bromo-uridine ou 5-bromo-déoxy-uridine. D'autres.groupes préférés susceptibles d'être mis en oeuvre dans le cadre de cette invention font intervenir des dérivés correspondants substitués par de l'iode ou du fluor, ou encore par des groupes $CF_3$.

Les sondes froides selon l'invention, qui peuvent, après hybridation, être ultérieurement détectées au moyen d'anticorps, de préférence des anticorps monoclonaux dirigés sélectivement contre les bases modifiées, présentent le net avantage qu'elles peuvent comporter un taux de substituton de l'une de leurs bases naturelles par des bases modifiées correspondantes, qui ne pouvait pas être atteint à ce jour dans la pratique, même lorsque les incorporations de ces bases modifiées dans un ADN bicaténaire par des techniques *in vitro*, par exemple semblables à celles envisagées dans la demande de brevet européen n° 0063879 sont mises en oeuvre, et ce sans affecter aucunement leur capacité d'hybridation (notamment appréciée par l'abaissement de température Tm) avec des séquences nucléotidiques complémentaires naturelles.

La sélectivité au niveau de la réaction antigène-anticorps, qui met en jeu chaque base modifiée individuellement, laquelle peut éventuellement dans certains cas - mais certainement pas tous les cas - être plus difficile à réaliser que dans le cas des sondes selon les techniques antérieures, est alors plus que largement compensée par le taux plus élevé, voire total des

substitutions de bases modifiées aux bases naturelles qui peuvent être réalisées, sans qu'il soit finalement porté le moindre préjudice à la capacité d'hybridation des sondes formées avec des ADN complémentaires.

Une classe de sondes conforme à l'invention, contenant un insérat tel qu'il a été défini plus haut, peut encore être définie par la structure :

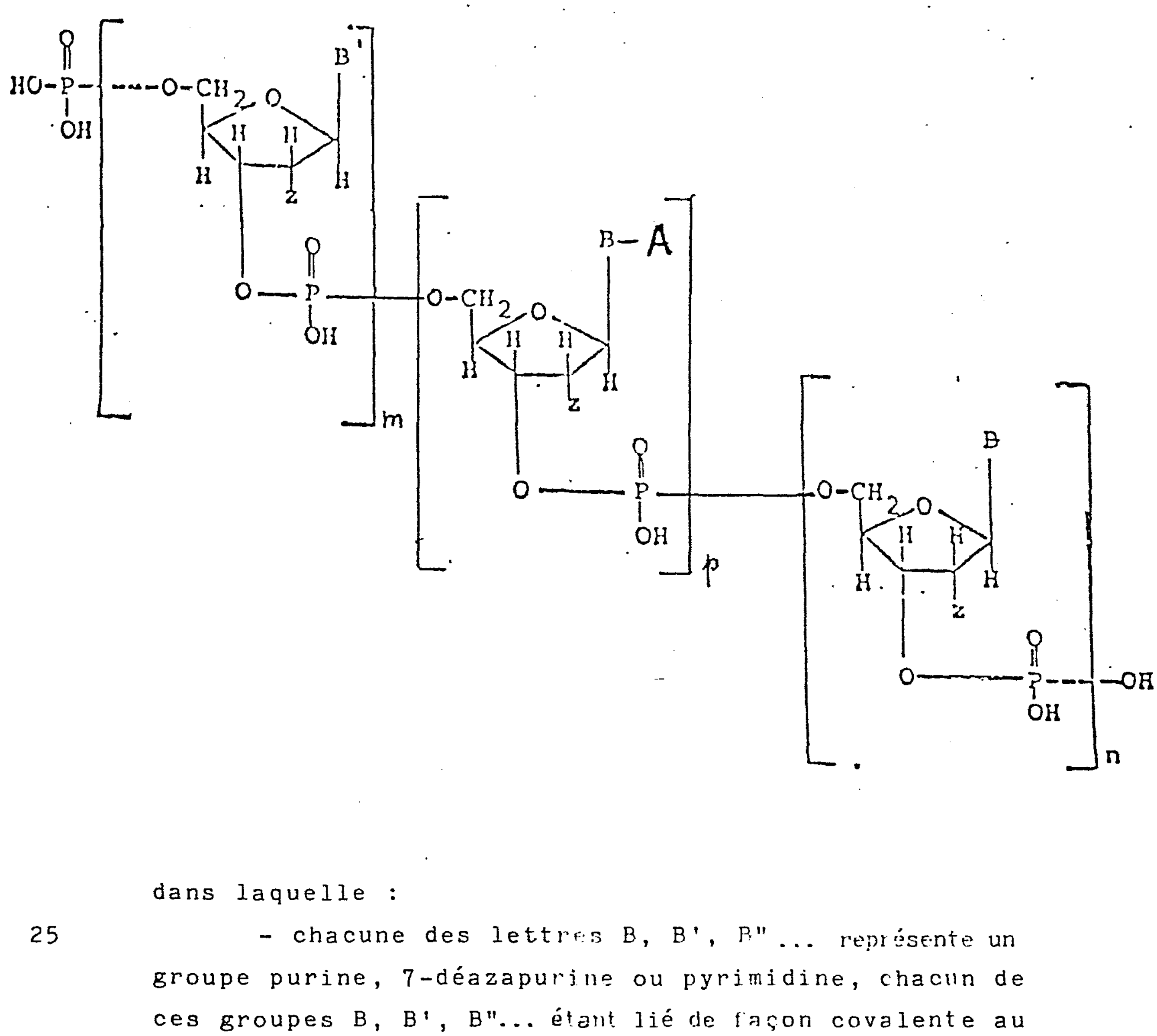

dans laquelle :

- chacune des lettres B, B', B" ... représente un groupe purine, 7-déazapurine ou pyrimidine, chacun de ces groupes B, B', B"... étant lié de façon covalente au groupe sucre correspondant en la position $C^1$ de celui-ci, soit au niveau de sa propre position $N^9$, pour les groupes purine ou 7-déazapurine, soit au niveau de sa

propre position $N^1$ pour les groupes pyrimidines,

- A représente un groupe de modifications de B, B', B" ....., selon le cas, consistant en un groupe de substitution comprenant au plus deux atomes de carbone et, de préférence, étant au surplus halogéné, ce groupe A étant directement lié à l'hétérocyle de la base azotée, soit en position 5, lorsque B, B', B" .... sont des groupes pyrimidine, soit en position 7, ou en position 8, ou dans les deux positions à la fois, lorsque B, B', B" ... sont des groupes déazapurine ou purine,

- z représente -H ou -OH et

- m et n représentent respectivement des nombres entiers de 0 à 100.000,

- p est un nombre entier de 1 à 75.000, étant entendu que la somme m + n + p est au moins égale à 2, de préférence au moins égale à 100.

Dans ce qui précède, l'expression "insérat" ne s'entend pas nécessairement comme signifiant qu'il s'agit d'une séquence d'acide nucléique hétérologue, c'est-à-dire d'origine étrangère, vis-à-vis des autres parties constitutives de la sonde froide. L'insérat et les autres parties constitutives peuvent être de même origine ou être dérivées d'un seul et même acide nucléique, par exemple lorsque la sonde est constituée par un fragment entier qui a été cloné dans un vecteur approprié, puis excisé de ce dernier, par exemple par l'intermédiaire d'une enzyme de restriction à laquelle ne correspondait aucun site de coupure dans le fragment d'ADN en cause. Cependant, il n'en sera pas de même dans de nombreux cas, en particulier chaque fois que la sonde sera en fait constituée par un plasmide ou cosmide recombinant, ou un ADN de phage recombinant contenant ledit insérat, surtout et évidemment lorsque celui-ci se trouvera être d'origine eucaryote.

On remarquera également que les modifications de l'une au moins des bases de la sonde, dans les conditions qui ont été spécifiées, peuvent ne pas affecter

la totalité de la sonde. Elles peuvent porter soit sur l'insérat lui-même, soit sur les autres parties de la sonde. Il pourra en être ainsi, chaque fois que la sonde aura été formée par recombinaison génétique in vitro, entre des fragments comportant des bases modifiées et des fragments distincts, dans lesquels les bases correspondantes n'auront pas été modifiées. Les modification conformes à l'invention pourront encore être concentrées sur une extrémité ou sur les deux extrémités opposées de la sonde, en particulier lorsque celle-ci (ou celles-ci) comportera (ou comporteront) un ou deux double-brin(s), tel(s) que poly-A-poly-U fixé(s) à cette (ou ces) extrémité(s) par l'intermédiaire d'une ARN-ligase ou d'une terminal-transférase appropriée, les bases soit du brin poly-A, soit du brin poly-U, pouvant alors être modifiées comme indiqué plus haut.

Parmi les sondes froides préférées de l'invention figurent celles dans lesquelles les bases modifiées mises en oeuvre ont été choisies parmi celles qui sont biologiquement incorporables dans l'ADN génomique d'un micro-organisme.

A cet égard, l'invention met à profit les constatations déjà faites par certains auteurs et selon lesquelles la déoxybromo-uridine (BrdUd) peut s'incorporer dans l'ADN des cellules eucaryotes à la place de la thymidine (Latt., PNAS U.S., 70, 3395(1974)). D'autre part, il a été décrit que les bases halogénées (5-bromo-uracile) par exemple pouvaient s'incorporer dans l'ADN des bactéries à la place de la thymine (ZAMENHOF et GRIBOFF, Nature 1954) et que le rendement d'incorporation était amélioré en utilisant une souche d'*E. coli* thymine-dépendante (HACKETT et al., Biochem. Biophys. Acta. 123 (1966)356-363). La préparation d'anticorps reconnaissant les bases brominées a été également décrite (SAWICKI et al. Science, 174, 70 (1971). Enfin des auteurs ont utilisé ces anticorps pour repérer la réplication de l'ADN dans les chromosomes ayant incorporé de la BrUd : GRATZNER et al. Exp. Cell. Res. 95, 88 (1975) ou même des anticorps monoclonaux (H.G GRATZNER, Science, vol. 218, 1982) .

Mais dans ces techniques, les souches de microorganismes obtenues, ayant incorporé BrdUd ne se sont en général pas révélées stables, ce qui, pour les applications envisagées, n'était d'ailleurs pas nécessaire.

Dans le cas des sondes froides dans lesquelles l'un au moins des types de nucléotides constitutifs de leur acide nucléique est remplacé par une base modifiée reconnaissable par les polymérases et comportant un groupe comprenant au plus deux atomes de carbone, il est particulièrement préféré que lesdits groupes comprenant au plus 2 atomes de carbone soient essentiellement toujours présents, en d'autres termes que les bases modifiées ne soient pas exemptes de ces groupes comprenant au plus deux atomes de carbone.

Dans le cas préféré dans lequel ce groupe comporte au moins un halogène ou est constitué par un halogène, il est donc préféré que la sonde froide soit essentiellement exempte de bases modifiées (qui remplacent l'un des types normaux de nucléotides constitutifs de l'acide nucléique) ne portant pas cet halogène ou ce groupe comportant cet halogène. Par exemple dans le cas de sondes dans lesquelles la thymine est au moins en partie remplacée par un groupe 5-halogèno-uracile, il est préféré qu'elles soient essentiellement dépourvues de groupes uracile libres. De préférence encore ces sondes sont essentiellement dépourvues de groupes pyrimidine reliés l'un à l'autre par des ponts C-C formés entre les positions 5 desdits groupes pyrimidine. On fera plus particulièrement référence au chapitre intitulé "Molecular mechanisms of radiation effects" de A. WACKER publié dans
pour ce qui est de la détection de la présence dans un ADN

des groupes sus-mentionnés. La présence de ces derniers du moins dans des proportions substantielles, altère très sensiblement la reproductibilité des essais d'hybridation auxquels les sondes selon l'invention sont destinées. D'une façon générale il est souhaitable que les sondes selon l'invention ou compositions les contenant soient exemptes de produits de photodécomposition.

Pour ces raisons il est préférable de maintenir les sondes à l'abri de la lumière, plus particulièrement à l'abri de radiations ayant des longueurs d'ondes inférieures à environ 400 nanomètres.

Les sondes selon l'invention peuvent être conservées à l'état lyophilisé, cependant de préférence alors dans des flacons ou récipients faits en une matière absorbant dans les longueurs d'ondes inférieures à 400 nanomètres. Les sondes selon l'invention peuvent également être conservées à l'état de solutions, soit dans un récipient répondant aux conditions sus-indiquées, soit dans des récipients dont les parois ne sont pas absorbantes, cas dans lequel ces solutions contiennent elles-mêmes un colorant inerte absorbant dans les longueurs d'ondes inférieures à 400 nm.

L'invention concerne également un procédé de fabrication <u>in vivo</u> de sondes du genre sus-défini dans des micro-organismes, notamment des bactéries, et plus particulièrement <u>E.coli</u>.

Le procédé selon l'invention pour fabriquer la sonde selon l'invention, lorsque celle-ci est dérivée d'un ADN recombinant ou d'un ADN-vecteur apte à transformer un microorganisme, tel que plasmide, cosmide ou ADN de phage et contenant l'insérat susdit, est caractérisé par la mise et le maintien en culture du microorganisme préalablement transformé par cet ADN recombinant avec un milieu de culture contenant la base modifiée en remplacement de la base naturelle correspondante, la susdite base modifiée s'y trouvant, soit à l'état libre, soit à l'état combiné,

dans un nucléoside ou nucléotide correspondant à ladite base, et par la récupération ultérieure de l'ADN-recombinant ou ADN vecteur.

De préférence, l'ensemble des opérations qui précèdent est réalisé à l'abri de radiations ayant des longueurs d'ondes inférieures à 400 nanomètres (ces radiations entraînant notamment la perte de l'halogène dans le cas d'acides nucléiques modifiés par des groupes 5-halogéno-uracyle). Notamment la préparation des sondes selon l'invention _in vivo_ et _in vitro_ est réalisée à l'obscurité ou à une lumière constituée de radiations ayant des longueurs d'onde supérieures à 400 nanomètres. L'introduction dans le milieu de culture des hôtes transformés par le vecteur porteur de l'acide nucléique (insérat) destiné à la préparation d'une sonde, d'un colorant inerte, non toxique pour l'hôte et le vecteur et absorbant dans les longueurs d'ondes inférieures à 400 nanomètres, constitue un moyen permettant de s'affranchir de la contrainte qu'imposent les restrictions qui précèdent quant à la nature de la lumière à utiliser. Par exemple le milieu sera teinté avec de l'éosine jaune. A titre d'exemple, on peut utiliser une solution d'éosine de telle manière que la concentration finale dans le milieu de culture soit 0,04% (poids/volume).

substitution *in vivo* de l'une au moins des bases contenues à l'intérieur d'acides nucléiques, notamment les susdits ADN-recombinants et ADN-vecteurs préalablement introduits dans le micro-organisme par des techniques du génie génétique, par des bases modifiées correspondantes telles que spécifiées ci-dessus.

De préférence, le micro-organisme initialement mis en oeuvre est une bactérie mutée, de préférence un mutant de *E. coli*.

D'une façon générale :

- la souche doit pouvoir accueillir, par exemple par transformation au moyen de plasmides (mais tous autres vecteurs, virus en particulier, doivent aussi pouvoir être utilisés) ou de DNA étranger ; elle doit donc essentiellement être "restriction -" ,
- la souche doit pouvoir incorporer un haut niveau de bases analogues des bases normales, et par conséquent l'anabolisme des bases normales doit être diminué ou supprimé, et le catabolisme des nucléotides (normaux ou anormaux,ou analogues) doit être fortement diminué ou supprimé, par des moyens biochimiques ou génétiques,
- la souche doit être stable et les vecteurs introduits dans la souche doivent l'être également. De préférence, ils sont aussi amplifiables, après incorporation dans leur séquence d'ADN de la base modifiée, analogue de la base naturelle.

Le caractère "restriction -" (restriction moins) est essentiel pour la mise en oeuvre directe du procédé préféré selon l'invention. Par mise en oeuvre directe, on entend sans passage préalble par une souche intermédiaire, elle-même restriction- (restriction moins). C'est ce caractère qui permet l'introduction et le maintien de certains acide nucléiques étrangers dans le micro-organisme. En l'absence de ce caractère, les vecteurs modifiés introduits dans le micro-organisme

seraient immédiatement hydrolysés par celles des enzymes de restriction, dont le rôle spécifique au sein du micro-organisme est de détruire les acides nucléiques étrangers introduits au sein de celui-ci.

L'incorporation *in vitro* des bases modifiées selon l'invention peut aussi se faire dans une souche bactérienne restriction + (restriction plus), dans la mesure où la préparation préalable du vecteur portant l'insérat qui doit subir l'incorporation de bases modifiées le permet, c'est-à-dire dans la mesure où ledit vecteur est insensible au système de restriction de l'hôte soit au moment du transfert dans l'hôte, soit au cours de sa multiplication dans l'hôte.

De préférence aussi la souche a été rendue auxotrophe pour la base naturelle correspondante. Mais cette auxotrophie pourrait être remplacée par toute autre mutation (ou par tous autres moyens biochimiques) ayant pour effets de renforcer la capacité du micro-organisme transformé à incorporer la base modifiée, par exemple de bloquer l'accès métabolique à la thymidilate synthétase ou au contraire d'inhiber tte enzyme.

De préférence, la base modifiée biologiquement incorporable est un dérivé de l'uracile ou de l'uridine, substituée en position 5 par un groupe comportant au plus deux atomes de carbone et, de préférence, halogéné, et en ce que le micro-organisme est de préférence auxotrophe pour la thymine ou pour les nucléosides ou nucléèotides correspondants. Plus particulièrement encore, le dérivé de l'uracile ou de l'uridine est un halogéno-uracile ou une halogéno-uridine, de préférence le bromo-uracile ou la bromo-uridine.

Dans ces derniers cas, le micro-organisme utilisé possède des mutations affectant au moins certains des gènes codant pour la synthèse de thymine endogène, sous le contrôle de la thymidylate synthétase, notamment une mutation dans le locus thyA.

On sait que de tels clones peuvent, notamment lorsqu'ils sont des mutants de E. coli, être sélectionnés en présence de triméthoprim, selon la technique décrite par MILLER et al. (1972, "Experiments in Molecular Genetics", Cold Spring Harbor Laboratory).

Une sélection supplémentaire permet de ramener l'auxotrophie à un niveau très faible, et en même temps permet une excellente incorporation finale de l'uridine halogénée, notamment de la bromo-uridine (BrU). Cette sélection repose sur l'observation que le métabolisme bactérien est orienté vers la dégradation des désoxyribonucléotides exogènes, dégradation qu'il faut prévenir. Toutes autres mutations du catabolisme, en particulier

celles qui sont associées à la répression catabolique (revue ULLMANN DANCHIN, 1983, Adv. Cyc. Nuc. Res. 15:1-53) pourront aussi être utilisées, avantageusement en association avec les mutations dont il est question ci-après et dont le rôle sera de ralentir, voire supprimer le catabolisme des souches de carbone. Cette sélection supplémentaire impliquera de préférence la réalisation de mutations dans les gènes contrôlant les dégradatiuons de désoxyribose-phosphates, notamment dans l'opéron deo (BACHMANN, K.B., 1980, 44:1-56, Microbiological Reviews, et MILLER (1972), en particulier dans le gène

deoB: qui contrôle la synthèse de la phosphopentomutase EC 2.7.5.6,et/ou dans le gène deoC qui contrôle la synthèse de la désoxyribose-phosphate aldolase EC 4.1.2.4). Une telle mutation s'obtient en sélectionnant à partir de la souche thyA qui requiert un haut niveau de thymine exogène (50-100 microgrammes par millilitre) des clones croissant à bas niveau (par exemple dans des milieux de culture contenant moins de 10 microgrammes/millilitre, notamment de l'ordre de 1 microgramme par millilitre au maximum).

Comme cela a déjà été mentionné plus haut, des souches préférées selon l'invention consistent en des mutants peu à même d'effectuer le catabolisme des sources de carbone, ces mutants étant notamment pourvus de mutations dans les locus (cya) codant pour l'adényl-cyclase ou dans le locus (crp) codant pour un récepteur de l'AMP cyclique ou dans les deux loci à la fois ; cela est utile pour permettre la présence à l'intérieur de la cellule d'un niveau élevé de désoryriboses phosphorylés, précurseurs des désoxyribonucléotides obtenus par incorporation de bases exogènes.

S'agissant de bactéries, de tels mutants peuvent être sélectionnés en mettant à profit la résistance connue de tels mutants à l'antibiotique connu sous la

marque "MECILLINAM" (Laboratoires LEO) et au phage lambda virulent. On peut sélectionner de telles souches en mettant en oeuvre les conditions opératoires suivantes :

100 microlitres d'une culture saturée de la souche initiale sont étalés en présence d'un nombre dix fois plus élevé d'un bactériophage lambda virulent, sur une boîte de Pétri contenant un milieu Mac Conkey Maltose (MILLER 1972), supplémenté avec la concentration léthale minimum pour E. coli de l'antibiotique. Cet antibiotique, en effet, permet une sélection efficace des mutants dépourvus d'adénylate cyclase (cya) ou de récepteur de l'AMP cyclique (crp) (R. AONO, M. YAMAZAKI, G. TAMURA (1979), J. Bact. 137:839-845) ; par ailleurs, les souches cya ou crp sont résistantes au phage lambda virulent (revue ULLMANN et DANCHIN 1983) et comme elles ne fermentent pas le maltose, elles apparaissent sous la forme de colonies blanches sur le milieu Mac Conkey.

Dans les modes de mise en oeuvre préférés du procédé selon l'invention, les micro-organismes utilisés comprennent simultanément l'ensemble de ces mutations. Elles comprendront de préférence encore, outre les mutations qui ont été mentionnées, celles qui affectent le système des phosphotransférases (système dépendant du phosphoénolpyruvate glycose:phosphotransférase: loci ptsHI crr en particulier, mais aussi systèmes associés, ainsi que d'autres systèmes liés à la répression catabolique (revue ULLMANN DANCHIN). La mutation cya peut être considérée comme contribuant fortement à la répression du catabolisme.

Il est à noter qu'un autre caractère essentiel préféré, particulièrement important, de la souche utilisée est la stabilité de son ADN. Il faut donc sélectionner des souches portant des mutations dans les gènes codant pour des exonucléases et endonucléases intervenant dans les systèmes de réparation de l'ADN, par exemple des mutations du type recB ou recC, ou les deux à la fois chez E. coli (exonucléase V) ; d'autres mutations affectant tant des exonucléases que des endonucléases, ainsi que le système de correction des erreurs de lecture au niveau de la réplication (système SOS) par exemple, peuvent être aussi utilisées, à la place ou en sus de celles qui se trouvent dans la souche décrite.

Les différentes opérations permettant de réaliser les susdites mutations, qu'il s'agisse de celles décrites dans la littérature ou de celles qui ont été rappelées plus haut (ou d'autres à la portée de l'expérimentateur spécialiste de cette technique) peuvent naturellement être réalisées dans un ordre quelconque, sous réserve des éventuelles imcompatibilités d'opérations de sélection entre elles, dès lors qu'elles seraient susceptibles d'interférences mutuelles au niveau du résultat recherché. Il va de soi que le spécialiste sera à même de faire les choix qui s'imposent quant à l'ordre le plus favorable des opérations de sélections successives à effectuer, selon la nature de la souche initiale qu'il choisira d'utiliser.

Avantageusement, on procèdera dans les conditions suivantes, notamment lorsque l'on aura recours à la souche initiale suivante connue pour l'efficacité avec laquelle elle peut être transformée et l'absence de système de restriction de l'ADN : souche C600 SF 8 (CA.STRUHL, JR. CAMERON, RW. DAVIS (1976), PNAS 73: 1471-1475).

Elle est traitée de manière à obtenir un mutant peu à même d'effectuer le catabolisme des sources de carbone exogène. Elle est soumise aux opérations de sélection dans les conditions décrites plus haut, en présence du phage lambda virulent et du "MECILLINAM" (4 microgrammes/ml) et de maltose (1% en volume).

Les souches cya ou crp sont résistantes au phage lambda virulent et ne fermentent pas le maltose apparaissant sous la forme de colonies blanches sur le milieu Mac Conkey. Au contraire, les colonies qui utilisent le maltose au cours de leur fermentation sont blanches. Un clone TP 6002, porteur de la mutation cya, stable, a été conservé. A partir de ce clone, on a isolé un mutant auxotrophe pour la thymine (thyA) par sélection en présence de triméthoprim (selon la méthode décrite par MILLER 1972), ce qui donne la souche, stable, TP 6030. Une sélection supplémentaire permet de ramener l'auxotrophie à un niveau très faible, lequel permet en même temps une excellente incorporation finale de BrU, par culture de cette souche, dans des milieux de plus en plus appauvris en thymine, jusqu'à atteindre un niveau de 1 microgramme par millilitre au maximum. Plusieurs clones ont été obtenus. Le clone TP 6033 a été utilisé par la suite en raison de sa stabilité.

Il est à noter qu'un autre caractère essentiel de la souche est la stabilité de son ADN, due à la présence des mutations recBrecC (exonucléase V) présentes dans la souche C600 SF 8 choisie entre autres pour cette raison, et à la présence des mutations affectant le catabolisme (cya en particulier).

La souche finalement obtenue peut être cultivée dans le milieu LB contenant, pour 1 litre, 10 grammes de Bacto tryptone et 5 grammes d'extrait de levure "BACTO YEAST EXTRACT", tous deux fabriqués par DIFCO, et 10 grammes de chlorure de sodium. Elle peut être conservée

dans le même milieu contenant en outre 8 % en volume de diméthyl sulfoxyde, du mannitol à une concentration finale de 0,1 % en poids et éventuellement de la superoxyde dismutase (SOD) issue de sang de boeuf, à raison de 2 microgrammes par millilitre (ou pour 3000 unités par milligramme de protéines). En général, la teneur en thymine apportée par le milieu LB est suffisante. Le cas échéant, il peut être complété avec 1 microgramme de thymine/ml.

Le procédé d'obtention d'une sonde froide conforme à l'invention peut également être utilisée pour la fabrication de sondes dans lesquelles ce n'est plus la thymine qui est substituée, au moins en partie par une base modifiée correspondante, mais une base distincte. L'application du procédé selon l'invention à ce dernier cas implique cependant la prise en compte d'une contrainte supplémentaire, à savoir une compartimentation appropriée entre les ribonucléotides et les désoxyribonucléotides, au sein des cellules en culture. En effet, la thymine, les ribonucléosides ou ribonucléotides dérivés de la thymine ne sont pas impliqués dans les grandes voies du métabolisme cellulaire. Tel n'est pas le cas pour les autres ribonucléosides. Il faut prévenir l'interconversion endogène entre ribonucléotides et désoxyribonucléosides, dans lesquels la base concernée serait également modifiée au niveau de la base correspondante. La compartimentation, naturelle pour la thymine, doit donc être imposée génétiquement pour les autres bases. Cela est possible par exemple en réalisant des mutations dans les gènes codant pour la ribonucléoside diphosphate réductase (EC 1.17.4.1.), notamment au niveau des loci nrdA et nrdB (BACHMANN loc. cit). Les mutants obtenus sont alors auxotrophes pour les désoxyribonucléosides.

Le procédé selon l'invention, tel qu'il a été défini plus haut pour fabriquer les sondes <u>in vivo</u> implique alors la présence dans le milieu de culture, non seulement de la base modifiée correspondante (à l'état libre ou combiné), mais également les autres désoxyribonucléosides (naturels ou également modifiés) dérivés de la base naturelle.

A titre d'exemple des bases modifiées distinctes de l'uracile, on peut mentionner la 5-bromocytosine, la 8-bromoguanine et la 8-bromo-adénine.

Les micro-organismes mis en oeuvre présenteront par ailleurs des mutations semblables à celles qui ont été évoquées. Les autres mutations conservent par ailleurs la même utilité, voire la même nécessité.

Le procédé préféré selon l'invention pour fabriquer des sondes marquées à un double avantage en ce qu'il permet, à partir d'un ADN-vecteur contenant l'insérat complémentaire de la séquence nucléotidique déterminée à détecter, la modification chimique <u>in vivo</u> et l'amplification simultanée de la sonde. Les techniques de transformation des micro-organismes, particulièremet des bactéries mutées, par les ADN recombinants contenant l'insérat à marquer, sont identiques à celles décrites dans la littérature, par exemple dans "Molecular Cloning, A Laboratory Manual" de T. MANIATIS et Coll.

Lorsque l'ADN recombinant est constitué par un plasmide contenant l'insérat à marquer, il est avantageux de réaliser la culture, en présence de la base modifiée, de préférence à une concentration de 0,5 à 500 et de façon encore plus préférée de 1 à 50 microgrammes par millilitre, la culture étant maintenue de préférence pendant au moins quatre générations et davantage si possible.

La purification des sondes amplifiées dans les micro-organismes récoltés au terme de la culture peut être réalisée de toute facon en soi connue. Il est avantageux de mettre en oeuvre la technique décrite par BIRNBOIM, H.C. et J. DOLY (1979, "A rapid alkaline extraction procedure for screening recombinant plasmid DNA", Nucleic Acids Res. 7:1513).

L'ouverture du plasmide et, s'il y a lieu, la séparation de la sonde et des autres parties du plasmide mettent égalemnt en jeu les techniques classiques du génie génétique, par exemple celles décrites dans "Molecular Cloning", "A Laboratory Manual" de T.MANIATIS, E.P. FRITSCH et J. SAMBROOK (C.S.H.).

La mise en oeuvre du procédé *in vivo* permet des substitutions, notamment de 25 %, dépassant avantageusement 75 % de la teneur en bases naturelles de la sonde traitée.

On peut même obtenir une substitution quasi totale de la base naturelle par la base modifiée, notamment en utilisant des concentrations élevées de la base modifiée dans le milieu de culture du micro-organisme.

Naturellement, les sondes selon l'invention peuvent également être construites en ayant recours à des méthodes de synthèse enzymatique ou chimique *in vitro*. En particulier, on peut utiliser la technique dite de "nick translation", par exemple dans les conditions décrites par RIGBY, P.W. et Coll. (1977, J. Mol. Biol. 113, 237-251), mais en substituant la bromodésoxyuridine triphosphate (BdU) à la thymidine triphosphate (TTP), aux mêmes concentrations. La sonde finalement obtenue contient une proportion de BdU dépassant 25 %. Les sondes obtenues peuvent être con-

servées au sein d'une solution tampon TE (Tris.Hcl. 10 mM, pH 8.0, EDTA 1 mM, pH 8.0).

Le procédé selon l'invention n'est pas limité à la fabrication de sondes conformes à la présente invention, c'est-à-dire dans lesquelles les bases modifiées comportent au plus deux atomes de carbone. Il peut être étendu à la production de sondes modifiées, dans lesquelles certaines au moins des bases naturelles sont remplacées par des bases résultant de la modification des précédentes, dans la mesure où lesdites bases modifiées n'interfèrent pas avec la capacité d'appariement WATSON-CRICK de l'acide nucléique modifié qui en résulte avec un acide nucléique complémentaire naturel ou contenant des bases modifiées semblables. A ce titre, le procédé selon l'invention s'étend à la fabrication de sondes, dans lesquelles les bases modifiées comportent des groupes de substitution, pouvant eux-mêmes comporter plus de deux atomes de carbone, par exemple des bases modifiées par la biotine. La condition essentielle à laquelle doivent naturellement également satisfaire ces bases modifiées est qu'elles soient biologiquement incorporables au sens que l'on a donné plus haut à cette expression. A ce titre, les bases, ribo- et désoxyribonucléosides et les ribo- et désoxyribonucléotides décrits dans la demande de brevet européen mentionnée plus haut, dès lors qu'ils satisfont également à la condition sus-indiquée.

L'invention concerne également les micro-organismes eux-mêmes qui sont particulièrement adaptés à la production de sondes in vivo, dans les conditions qui ont été indiquées.

En particulier, elle concerne les micro-organismes, de préférence du type bactérie, telle que E. coli, caractérisés par une combinaison de mutations ou propriétés :

- ils sont de préférence restriction - (restriction moins),
- mutations affectant certains au moins des gènes, de préférence tous les gènes, contrôlant la synthèse endogène de l'une des bases intervenant dans la constitution des nucléosides naturels,
- mutations affectant certains au moins des gènes contrôlant le catabolisme des sources de carbone et des désoxyriboses intracellulaires,
- mutations affectant certains au moins des gènes, et de préférence tous les gènes contrôlant la synthèse des nucléases affectant l'ADN interne.

Elle concerne également les susdits micro-organismes hébergeant un vecteur recombinant contenant, le cas échéant, un insérat dans le cas où ce vecteur ou des parties de celui-ci sont destinés à être utilisés ultérieurement en tant que sonde, ces micro-organismes comportant également une combinaison de mutations affectant simultanément :

- certains au moins des gènes, de préférence tous les gènes contrôlant la synthèse endogène de l'une des bases intervenant dans la constitution des nucléosides naturels,
- certains au moins des gènes contrôlant le catabolisme des sources de carbone et des désoxyriboses intracellulaires,
- certains au moins des gènes et de préférence tous les gènes contrôlant la synthèse des nucléases affectant l'ADN interne.

Des micro-organismes (transformés ou non) préférés sont caractérisés par une combinaison de mutations affectant simultanément les loci thyA, cya ou crp ou les deux à la fois, deo, recB, recC.

Le procédé selon l'invention fournit donc un moyen permettant le renouvellement aisé, et à coût relativement faible, de toutes sondes ou plus généralement encore de tous ADN recombinants pouvant être reconnus par des techniques de marquage, par exemple selon les méthodes qui ont été envisagées, par exemple dans la demande de brevet européen n° 0063869-A3 et dont des modes de mise en oeuvre préférés seront encore rappelés plus loin. De préférence, la détection est néanmoins réalisée par le biais d'anticorps polyclonaux ou, de préférence, monoclonaux. De ce même point de vue, les souches selon l'invention transformées par un ADN recombinant (non encore marqué lui-même) peuvent être maintenues en culture ou, le cas échéant, à l'état lyophilisé. Lorsque les souches en question sont auxotrophes pour l'une des bases susmentionnées, il convient naturellement de les maintenir (pour le cas où elles ne sont pas à l'état lyophilisé) dans un milieu de conservation contenant de faibles doses de la base naturelle. Elles peuvent par conséquent, à la demande, servir de source de vecteur modifié par un insérat - cas de la sonde froide - ou non modifié, par mise en contact ou en culture dans un milieu contenant en lieu et place de la base naturelle correspondante une base modifiée biologiquement incorporable. Les sondes elles-mêmes sont de préférence conservées à l'abri de radiations ayant des longueurs d'onde inférieures à 400 nm.

L'invention concerne encore des ADN-vecteurs, notamment plasmides, cosmides ou ADN de bactériophages, dans lesquels l'une au moins des bases est au moins en partie substituée par une base modifiée, dans laquelle le groupe de modification comprend au plus deux atomes de carbone, le cas échéant halogéné, ou est constitué par un halogène.

Dans le cas où ces recombinants constituent des sondes au sens indiqué plus haut, c'est-à-dire qu'ils

sont modifiés par un insérat complémentaire d'une séquence déterminée de nucléotides qui est à rechercher dans des échantillons biologiques, ils peuvent être mis en oeuvre dans toutes les applications qui ont été exposées dans la demande de brevet européen sus-indiquée, que ce soit pour des applications, par exemple de diagnostic médical, de cartographie de gènes, de réalisation de caryotypes, de détection d'anomalies génétiques, d'identification ou de détection de micro-organismes spécifiques.

Les vecteurs selon l'invention peuvent, à l'instar des vecteurs "normaux" pour ce qui est de la constitution des nucléotides entrant dans leurs acides nucléiques, être utilisés comme vecteurs de clonage. Ils peuvent être excisés par des enzymes de restriction et recombinés avec d'autres séquences nucléiques, en mettant en oeuvre les endonucléases ou ligases classiques selon la nature de l'opération réalisée, en d'autres termes être utilisés dans des techniques du génie génétique.

Toutes ces opérations sont de préférence effectuées à l'abri de radiations ayant des longueurs d'onde inférieures à 400 nm. Les vecteurs peuvent en outre être utilisés comme marqueurs de sélection, grâce aux bases modifiées qu'ils contiennent. A cet égard ils sont d'une application particulièrement intéressante lorsqu'il s'agit de détecter celles des cellules (procaryotes ou eucaryotes) dans lesquelles ils auront été introduits, le cas échéant avec un insérat particulier dont l'expression dans les cellules est recherchée.

Ce marquage est d'autant plus intéressant que la détection des bases modifiées, en particulier par des anticorps sélectivement dirigés contre les bases modifiées, n'implique pas nécessairement l'extraction préalable des acides nucléiques provenant des cellules transformées. La détection peut être réalisée sur des cellules entières, même intactes.

De préférence les anticorps servant à la détection sont monoclonaux. Ils se comportent comme s'ils pénétraient à l'intérieur des cellules et ils peuvent à leur tour, être reconnus par d'autres anticorps dirigés

contre eux ou par des molécules affines distinctes, ces autres anticorps ou molécules affines portant par exemple le groupe enzymatique ou fluorescent permettant leur détection par des méthodes classiques.

On décrit ci-après un exemple de préparation d'anticorps monoclonaux dirigés contre la bromo-uridine.

Des anticorps monoclonaux appropriés à la reconnaissance du nucléotide modifié préféré, notamment par la iodouridine, peuvent être préparés comme indiqué ci-après.

Des nucléotides halogénés (BrUd, iodouridine) ont été couplés à des protéines (albumine et ovalbumine), afin de les rendre immunogéniques. Ce couplage a été effectué par un procédé qui consiste à oxyder le ribose du nucléotide par le périodate de sodium ; les fonctions aldéhydes ainsi créées réagissent dans un deuxième temps avec les groupements aminés de la protéine (ERLANGER et BEISER, PNAS U.S., 52, 68 (1964)). La substitution a été environ de 5 à 15 molécules de nucléotides par molécule de protéines (support).

Des souris issues d'un croisement entre des BALB/C et C57BL/6 ont été immunisées avec l'iodouridine couplée à l'ovalbumine émulsionnée dans les substances adjuvantes de Freund complètes à raison de 200 microgrammes par souris et par injection. Les souris ont reçu deux à trois injections à un mois d'intervalle. Les cellules de la rate et des ganglions poplités d'une souris ayant reçu au préalable une injection de rappel ont été fusionnées au myélome murin SP20 par l'intermediaire du polyéthylène glycol d'après la technique

décrite par KÖHLER et MILSTEIN, Nature, 256, 495 (1975) et modifiée par GALFRE et al., Nature, 266, 550 (1977). Les surnageants des puits contenant des clones cellulaires ont été testés par une méthode immunoenzymatique : l'albumine couplée soit à la BrUd ou à la méthyl-uridine (analogue de la thymidine) a été fixée à des plaques de polystyrène. Les plaques sont incubées avec les surnageants puis avec un anti-Ig de souris couplé à la bêta-galactosidase. Les clones reconnaissant uniquement l'albumine-BrUd l'albumine-thymidine ont été sélectionnés, clonés et les cellules injectées à des souris de façon à obtenir des ascites riches en anticorps monoclonaux. Les anticorps ont été isolés de ces ascites sur une colonne de protéine-A couplée au Sépharose. Les anticorps purifiés ont été testés quant à leur reconnaissance uniquement de la BrUd et non de la thymidine sur des noyaux de cellules de mammifères (fibroblastes) cultivées en présence ou non de BrdUd par une technique immunoenzymatique.

Un hybridome secréteur de tels anticorps a été déposé le 23 novembre 1983 à la Collection Nationale de Culture de Micro-Organismes de l'Institut Pasteur de Paris (C.N.C.M.). Il lui a été attribué le n° I-263.

La mise en contact de ces anticorps monoclonaux avec la sonde ou l'ADN-vecteur comportant des bases BrUd peut être réalisée en mettant en jeu des techniques classiques.

On peut notamment réaliser ce contact après immobilisation de l'ADN simple brin ou double brin (provenant de l'échantillon à étudier, par exemple de fibroblaste) sur un filtre de nitrocellulose (ou tout autre support approprié). Avantageusement, la révélation sera ensuite réalisée par une méthode immunocytochimique comportant : l'incubation des filtres avec des anticorps monoclonaux murins anti-ADN, lavage, incubation avec

des anticorps anti-immunoglobulines de souris marqués avec la péroxydase, lavage, coloration histochimique de l'enzyme. Cette technique permet de mettre en évidence jusqu'à 0,1 pg d'ADN ayant incorporé de la BrU, déposé sur le filtre de nitrocellulose, tandis qu'aucune coloration n'est observée avec l'ADN "naturel" correspondant. Les résultats obtenus indiquent que les conditions expérimentales utilisées permettent d'obtenir des sensibilités de détection performantes sans qu'aucun système d'amplification ait été utilisé. L'absence de bruit de fond permet d'envisager une amplification de sensibilité pour le moins d'un facteur 10.

On peut naturellement avoir recours à toute méthode de détection connue. Sont particulièrement avantageuses les méthodes décrites dans les articles dont les références suivent, notamment en rapport avec les questions suivantes :

- Détection immunocytochimique de la péroxydase couplée à des anticorps présents dans des cellules :
AVRAMEAS (Immunochem., 1969, vol. 6, p. 825) ;
- Détection de la péroxydase non couplée, présente dans des tissus :
GRAHAM et al (Histochem. and Cytochem., 1965, vol. 13, p. 150) ;
- Utilisation de la bêta-galactosidase comme marqueur dans un dosage immunoenzymatique :
GUESDON, THIERRY, AVRAMEAS (J. Allergy Clin. Immunol., 1978, vol. 61, p. 23).

Des compléments d'information relatifs à l'invention apparaîtront encore au cours de la description des conditions dans lesquelles les différentes opérations peuvent être réalisées. Bien entendu, elles n'ont pas un caractère limitatif.

On a recours à la souche de E. coli, et plus particulièrement du clone TP 6033, lequel a été préalablement transformé par un ADN-vecteur quelconque, par exemple par les techniques décrites dans "Molecular Cloning, A Laboratory Manual" de T. MAGNATIS et Coll., dans la mesure où ceux-ci décrivent des procédures de transformation de E. coli.

Cette transformation a été réalisée sur la susdite souche dans un milieu de culture contenant la thymine naturelle.

CULTURE DE LA BACTERIE EN VUE D'INCORPORATION DE 5-BU (5-bromo-uridine)

La bactérie est cultivée en milieu 63 B1 ou milieu minimum supplémenté en glucose, casaminoacides et en adénine (0,1 mg/ml) et thymine (0,1 à 1 microgramme/ml). Les antibiotiques nécessaires au plasmide spécifique inséré dans la souche sont apportés au milieu de culture.

Après ensemencement de l'un de ces milieux servant à la préculture de la bactérie et culture à 37°C jusqu'à une DO (densité optique à 600 nm) de 1,2 à 2, un aliquot est prélevé et transféré (DO finale de 0,1 à 0,3) dans le milieu concerné, sans thymine mais supplémenté en 5-bromo-uracile (1 à 100 microgrammes/ml) dans les mêmes conditions. La culture est maintenue pendant deux générations au minimum et le plasmide purifié par les techniques classiques (BIRNBOIM et DOLY. NAR 7, 1513). A titre d'exemple, une culture de quatre générations, en présence de 1 microgramme/ml en 5-bromo-uracile, avec une concentration en thymine résiduelle au plus de 0,1 microgramme/ml, permet d'obtenir 25 % de substitution de la thymidine incluse dans le plasmide incorporé.

I - DEPOT DU DNA CIBLE SUR FILTRE

Des filtres de nitrocellulose BA-85 (SCHLEICHER et SCHUELL) ont été utilisés.

Pour les dépôts en petites taches "Dot-blots",

le DNA est dilué dans du TE ou du TBS (Tris HCl. 20; mM pH 7,8, NaCl 0,15 M) contenant 1,5 mg/ml de DNA de sperme de saumon (DNA carrier) soniqué, dénaturé thermiquement (100°C pendant 5-10 minutes, puis trempé dans de la glace en surfusion) préalablement ou non. Le DNA cible est prédénaturé suivant la même technique. Les filtres, séchés à l'air, sont ensuite cuits à 80°C dans un four à vide, durant 2 heures.

Le transfert depuis les gels d'agarose (séparation électrophorétique) sur les filtres BA 85, est effectué suivant la technique de THOMAS (P.S. 1980 ; DNAS 77, 5201-5205); d'après SOUTHERN (C.M. 1975, J. Mol. Biol. 98, 503-517).

II - PREHYBRIDATION

Le tampon de préhybridation est celui décrit par LEARY J.L. et Coll. 1983 ("Rapid and sensitive colorimetric method for visualising biotin-labelled DNA probes hybridized to DNA or RNA immobilized on nitrocellulose": Bio-blots - PNAS 80, 4045-4049, d'après WAHL et Coll. 1979 PNAS 76, 3683-3687), dans lequel le DNA de sperme de hareng soniqué est remplacé par du DNA de sperme de saumon à 250 microgrammes/ml ; la présaturation protéique nécessaire à la révélation immunoenzymologique est effectuée conjointement par ajout de sérum normal (SN) de mouton ou de chèvre décomplémenté (10 % v/v) et de sérum albumine de boeuf (BSA) à 1 %.

L'incubation a lieu à 42°C pendant 2 heures, à raison d'environ 1 ml pour 10 $cm^2$ de filtre ; elle est réalisée dans un "sac scellé" (scellobag) avec une légère agitation.

Le tampon est retiré pour être remplacé par le tampon d'hybridation.

III - HYBRIDATION

La sonde-BU est dénaturée à 100°C (5-10 minutes), puis refroidie brutalement (glace). L'incubation (2 à 9 heures) a lieu comme ci-dessus avec un tampon d'hybridation contenant 45 % de formamide déionisée, 250 microgrammes/ml de DNA de sperme de saumon soniqué (J.L. LEARY et Coll., 1983, "Rapid and sensitive colorimetric method ..." Bio-blots. PNAS, 80, 4045-4049) et supplémentée en sérum normal/BSA (10 %/1 %) comme précédemment. Les lavages sont conformes à J.L. LEARY et Coll. (1983, "Rapid and sensitive colorimetric method .." Bio-blots PNAS, 80, 4045-4049) pour une hybridation à 45 % de formamide déionisée.

Les filtres sont séchés à l'air.

IV - DENATURATION DES ZONES HYBRIDEES

Les anticorps anti-BU reconnaissant mieux le DNA simple brin que double brin, une dénaturation sur filtre est effectuée. Les filtres sont trempés 15 à 30 secondes dans NaOH 0,5 M, NaCl 1,5 M, puis 5 à 10 minutes dans une solution de neutralisation (Tris HCl 0,5 M, pH 8,0, NaCl 1,5 M). Les filtres sont ensuite séchés à température ambiante.

V - REVELATION IMMUNOENZYMOLOGIQUE

Les filtres sont prémouillés avec du TBS + TWEEN (0,1 %) (TBS.T), puis placés dans des "scellobags". L'anticorps (souris) anti-BU est ajouté dans du TBST avec 10 % de S N décomplémenté de cheval ou de mouton et 1 % de BSA à raison de 1 ml par $cm^2$ de filtre. L'incubation est effectuée, sous agitation douce, 2 heures à 37°C ou une nuit à température ambiante et à des concentrations d'anticorps de 1 microgramme/ml à 20 microgrammes/ml.

Trois rinçages de 15 minutes avec du TBST sont effectués (agitation légère) à température ambiante.

Un anticorps de mouton, dirigé contre les gammaglobulines de souris et couplé à la péroxidase, est ensuite ajouté, dans les mêmes conditions (1 à 5 microgrammes/ml) et laissé en contact avec les filtres durant 2 heures à température ambiante. Les filtres sont ensuite

rincés comme ci-dessus.

La présence de la péroxydase est révélée au moyen du substrat DAB (Diamino-benzidine) donnant un produit insoluble.

VI - RESULTATS (chiffres exprimés en pg de DNA présents sur le filtre). Des sensibilités de détection de 0,5 à 5 pg de DNA-BU dénaturé (respectivement marqués *in vivo* ou par "Nick-translation")sont obtenues par cette méthode en "Dot-blot".

Une détection de l'ordre de 50 pg de DNA est assurée, après hybridation.

Ces valeurs sont déjà, en l'absence de toute amplification des signaux ou optimisation des systèmes, au niveau des systèmes,les plus performants.

Les souches suivantes ont été déposées à la C.N.C.M. le 22 novembre 1983 :

- souche *E. coli* TP 6002 sous le n° I-259,
- " " " TP 6030 " " n° I-260,
- " " " TP 6033 " " n° I-261.

## REVENDICATIONS

1 - Procédé d'obtention d'une sonde froide pour la détection par hybridation d'une séquence déterminée de nucléotide et qui comporte elle-même un insérat comprenant une séquence complémentaire de ladite séquence déterminée de nucléotides, dans laquelle au moins une partie des bases d'au moins l'un des types de nucléotides constitutifs de son acide nucléique est remplacée par des bases modifiées reconnaissables par les polymérases, ladite base modifiée étant biologiquement incorporable dans l'ADN génomique d'un microorganisme et n'interférant pas avec la capacité d'appariement WATSON-CRICK de l'acide nucléique modifié qui en résulte avec un acide nucléique complémentaire naturel ou contenant des bases modifiées semblables, ce procédé étant caractérisé par la mise et le maintien en culture du microorganisme préalablement transformé par une sonde dans laquelle la base naturelle correspondante n'est pas modifiée, avec un milieu de culture contenant la base modifiée en remplacement de la base naturelle correspondante, la susdite base modifiée s'y trouvant, soit à l'état libre, soit à l'état combiné, dans un nucléoside ou nucléotide correspondant à ladite base, et par la récupération ultérieure de l'ADN-recombinant ou ADN-vecteur à partir de la culture dudit microorganisme

2 - Procédé selon la revendication 1 caractérisé par le fait qu'il est mis en oeuvre dans des conditions assurant la mise de la sonde à l'abri de radiations ayant des longueurs d'onde inférieures à 400 nanomètres.

3 - Procédé selon la revendication 2,caractérisée en ce que la base modifiée comporte un groupe comprenant au plus deux atomes de carbone et, de préférence, au moins un halogène, ou un groupe constitué par un halogène.

4 - Procédé selon la revendication 3, caractérisé en ce que le microorganisme est un mutant de bactérie, de préférence le E.coli.

5 - Procédé selon l'une quelconque des revendications 1 à 4, caractérisé en ce que le microorganisme est ou a été rendu auxotrophe pour la base naturelle correspondant à la susdite base modifiée.

6 - Procédé selon la revendication 5, caractérisé en ce que la base modifiée est un dérivé de l'uracile ou de l'uridine, substituée en position 5 par un groupe comportant au plus deux atomes et, de préférence, halogéné et en ce que le microorganisme est auxotrophe pour la thymine ou pour les nucléosides correspondants.

7 - Procédé selon la revendication 5, caractérisé en ce que la base modifiée est un halogéno-uracile ou une halogéno-uridine, de préférence le bromo-uracile ou la bromo-uridine.

8 - Procédé selon l'une quelconque des revendications 1 à 4, caractérisé en ce que le microorganisme possède une mutation affectant le ou les gènes codant pour la synthèse endogène de la thymine endogène, sous le contrôle de la thymidylate synthétase, notamment une mutation dans le locus thyA.

9 - Procédé selon l'une quelconque des revendications 1 à 4, caractérisé en ce que la base biologiquement modifiée est dérivée d'une base naturelle distincte de la thymidine ou de l'uracile ou d'un nucléoside correspondant à cette base distincte, en ce que le microorganisme est auxotrophe pour les désoxyribonucléosides et en ce que la culture est réalisée en présence non seulement de la base modifiée correspondante (à l'état libre ou combiné), mais également des désoxyribonucléosides dérivés des autres bases naturelles.

10 - Procédé selon la revendication 9, caractérisé en ce que ledit microorganisme comporte les gènes codant pour la ribonucléoside diphosphate réductase, notamment dans le locus nrdA, ou dans le locus nrdB, ou de préférence dans les deux loci à la fois.

11 - Procédé selon la revendication 10, caractérisé en ce que la souche est un mutant peu à même d'effectuer le catabolisme des sources de carbone, ce mutant étant notamment pourvu de mutations dans le locus (cya) codant pour l'adénylcyclase, ou dans le locus (crp) codant pour un récepteur de l'AMP cyclique ou dans les deux à la fois.

12 - Procédé selon la revendication 11, caractérisé en ce que le susdit microorganisme comprend aussi des mutations dans les gènes affectant la synthèse des phosphotransférases, notamment dans les loci ptsHI crr.

13 - Procédé selon la revendication 12, caractérisé en ce que le susdit microorganisme comprend aussi des mutations dans les opérons contrôlant la dégradation des désoxyribose phosphates, notamment les opérons (deo) et plus particulièrement dans l'opéron (deoB) ou dans l'opéron (deoC) ou dans les deux opérons à la fois.

14 - Procédé selon la revendication 13, caractérisé par des mutations dans des gènes codant pour des exonucléases et endonucléases intervenant dans les systèmes de réparation de l'ADN, telles que des mutations du type recB ou recC, ou les deux à la fois, notamment lorsque le microorganisme mis en oeuvre est E.coli.

15 - Microorganisme, de préférence du type bactérie, tel que E.coli, pour la mise en oeuvre du procédé selon l'une quelconque des revendications 1 à 14,caractérisé par une combinaison de mutations ou propriétés :

- il est "restriction moins",
- mutations affectant certains au moins des gènes, de préférence tous les gènes contrôlant la synthèse endogène de l'une des bases intervenant dans la constitution des nucléosides naturels,
- mutations affectant certains au moins des gènes contrôlant le catabolisme des sources de carbone et des désoxyriboses intracellulaires,
- mutations affectant certains au moins des gènes, et de préférence tous les gènes contrôlant la synthèse des nucléases affectant l'ADN interne.

16 - Microorganisme selon la revendication 15, caractérisé par une combinaison de mutations affectant simultanément les loci thyA, cya ou crp ou les deux à la fois, deo, recB, recC.

17 - Microorganisme selon la revendication 15 caractérisé par le fait qu'il héberge un vecteur recombinant .

18 - Microorganisme selon la revendication 17, caractérisé par une combinaison de mutations affectant simultanément un locus contrôlant la synthèse endogène d'une base naturelle distincte de la thymine, et les loci cya, crp, deoB , deoC, recBc, nrdA et nrdB.

19 - ADN recombinant formant une sonde froide pour la détection par hybridation d'une séquence déterminée de nucléotides et comportant elle-même un insérat comprenant une séquence complémentaire de ladite séquence de nucléotides, caractérisée en ce qu'au moins une partie des bases d'au moins l'un des types de nucléotides constitutifs de son acide nucléique est remplacée par des bases modifiées reconnaissables par les polymérases et comportant un groupe de substituts comprenant au plus deux atomes de carbone, ladite sonde froide étant essentiellement exempte de bases modifiées du genre sus-indiqué, dépourvues du susdit groupe de substitution.

20 - ADN-recombinant selon la revendication 19, caractérisé en ce que ce groupe comporte au moins un halogène ou est constitué par un halogène.

21 - ADN-recombinant selon la revendication 19 caractérisé en ce qu'il-est homologue d'un vecteur réplicable dans un microorganisme, tel qu'ADN de phage ou plasmide, modifié par le susdit insérat.

22 - ADN-recombinant selon l'une quelconque des revendications 19 à 21,caractérisé en ce que les bases modifiées sont choisies parmi celles qui sont susceptibles de s'incorporer biologiquement dans l'ADN génomique d'un microorganisme, notamment de <u>E.coli</u>.

23 - ADN-recombinant selon la revendication 19 , caractérisé en ce que les bases modifiées sont dérivées de bases des nucléotidiques naturelles dont la modification réside dans une substitution par le susdit groupe, en position 5, lorsqu'il s'agit d'une base pyrimidine, et en position 7 ou en position 8, ou dans les deux positions à la fois, lorsqu'il s'agit d'une base déazapurine ou purine.

24 - ADN-recombinant selon la revendication 19 , caractérisé en ce que les bases modifiées sont des groupes 5-halogéno-uracile, de préférence 5-bromo-uracile.

25 - ADN-recombinant selon la revendication 19 ,caractérisée par la structure

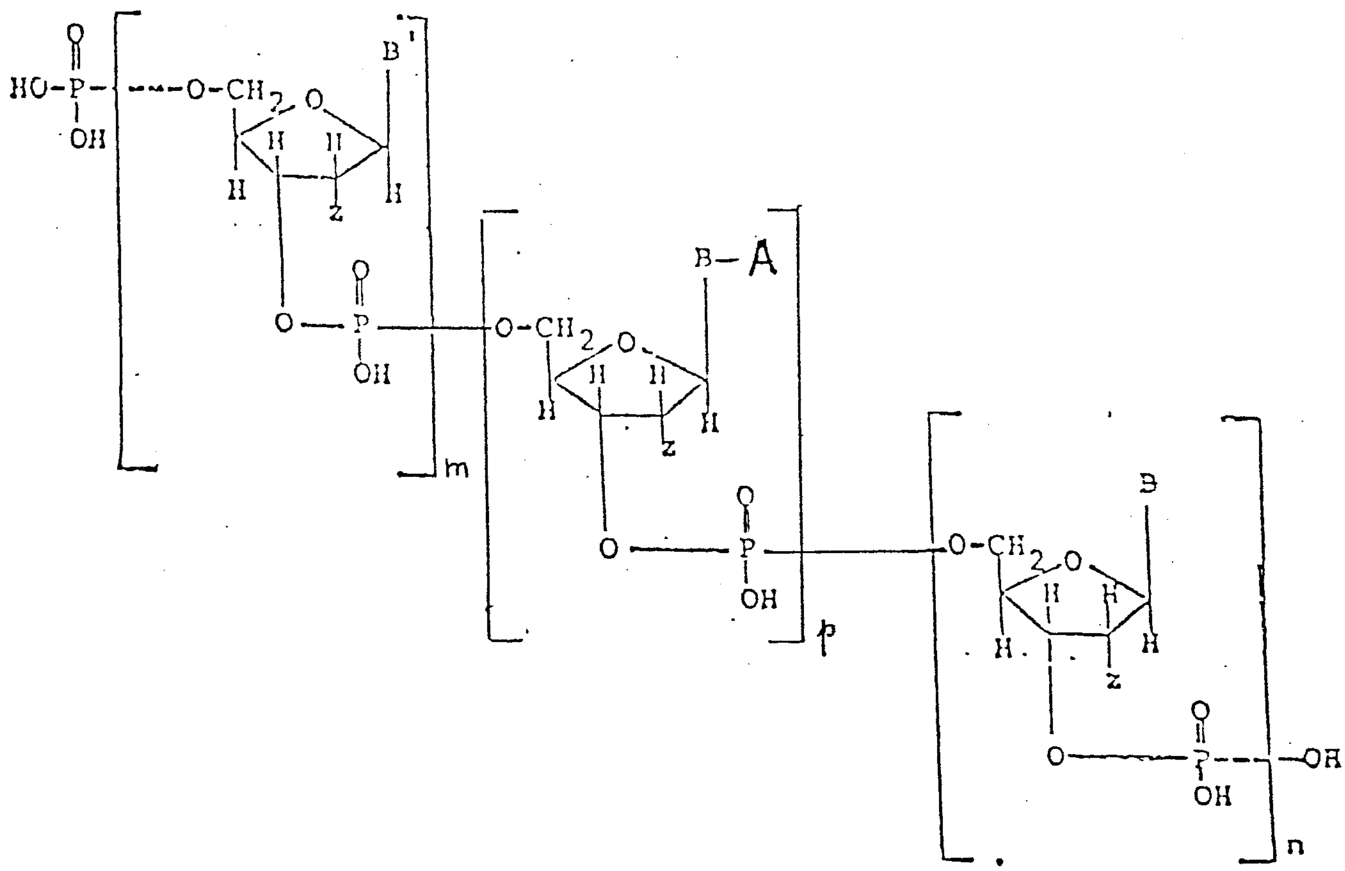

dans laquelle :

- chacune des lettres B, B', B"... représente un groupe purine, 7-déazapurine ou pyrimidine, chacun de ces groupes B, B', B"... étant lié de façon covalente au groupe sucre correspondant en la position $C^1$ de celui-ci, soit au niveau de sa propre position $N^9$, pour les groupes purine ou 7-déazapurine, soit au niveau de sa propre position $N^1$ pour les groupes pyrimidine,
- A représente un groupe de modifications de B, B', B"... selon le cas, consistant en un groupe de substitution comprenant au plus deux atomes de carbone et, de préférence, étant au surplus halogéné, ce groupe A étant directement lié à l'hétérocyle de la base azotée, soit en position 5, lorsque B, B', B"... sont des groupes pyrimidine, soit en position 7, ou en position 8, ou dans les deux positions à la fois, lorsque B, B', B"... sont des groupes déazapurine ou purine,
- z représente -H ou -OH et
- m et n représentent respectivement des nombres entiers de 0 à 100 000,
- p est un nombre entier de 1 à 75 000, étant entendu que la somme m + n + p est au moins égale à 2, de préférence au moins égale à 100.

26 - ADN-recombinant selon l'une quelconque des revendications 19 à 25, caractérisé en ce que les substitutions de bases modifiées aux bases naturelles sont dans une proportion d'au mçins 25 %, de préférence dépassant 75 % de la teneur totale en bases naturelles correspondantes de ladite sonde.

- 27 Procédé pour détecter un acide nucléique contenant la susdite séquence déterminée de nucléotides, caractérisé par la mise en contact de cet acide nucléique avec la sonde froide selon la revendication 19, soit seule, soit combinée avec l'une quelconque des revendications 20 à 26 dans des conditions permettant leurs dénaturations respectives préalables, s'il en est besoin, et leur hybridation mutuelle, la séparation de la sonde en excès n'étant pas intervenue dans ladite hybridation - les opérations qui précèdent étant de préférence réalisées à l'abri des radiations ayant des longueurs d'onde inférieures à 400 nm - et la détection, directe ou indirecte de l'hybride éventuellement formé, notamment par réaction avec un anticorps dirigé contre les bases modifiées de la sonde, celui-ci pouvant, le cas échéant, à son tour être détecté par un autre anticorps ou une autre molécule affine pour le premier anticorps.

Office européen des brevets

**0143059**
Numéro de la demande

## RAPPORT DE RECHERCHE EUROPEENNE

EP 84 40 2392

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | ANN. IMMUNOL. (INSTITUT PASTEUR), vol. 134D, 1983, pages 399-405; F. TRAINCARD et al.: "Une technique immunoenzymatique pour la mise en évidence de l'hybridation moléculaire entre acides nucléiques" * En entier * | 1,19-27 | C 12 Q 1/68<br>C 12 N 15/00<br>C 07 H 21/04<br>C 12 N 1/20 //<br>(C 12 N 1/20<br>C 12 R 1:19 ) |
| D,A | EP-A-0 063 879 (YALE UNIVERSITY) | | |
| D,A | SCIENCE, vol. 218, no. 4571, 29 octobre 1982, pages 474-475; H.G. GRATZNER: "Monoclonal antibody to 5-bromo- and 5-iododeoxyuridine: A new reagent for detection of DNA replication" | | **DOMAINES TECHNIQUES RECHERCHES (Int. Cl.4)** |
| A | CHEMICAL ABSTRACTS, vol. 98, no. 21, 23 mai 1983, page 36, no. 172644c, Columbus, Ohio, US; L. FOX et al.: "Incorporation of 5-substituted analogs of deoxycytidine into DNA of herpes simplex virus-infected or -transformed cells without deamination to the thymidine analog" & ANTIMICROB. AGENTS CHEMOT HER. 1983, 23(3), 465-476 | | C 12 Q<br>C 12 N<br>C 07 H |
| | --- -/- | | |

Le présent rapport de recherche a été établi pour toutes les revendications

| Lieu de la recherche | Date d'achèvement de la recherche | Examinateur |
|---|---|---|
| LA HAYE | 26-02-1985 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

**RAPPORT DE RECHERCHE EUROPEENNE**

**0143059**

Numéro de la demande

EP 84 40 2392



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | WO-A-8 302 286 (INSTITUT PASTEUR)<br>----- | | |
| | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 26-02-1985 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82